# FASCICULE DE BREVET EUROPEEN

(11) **EP 1 753 360 B1**
(45) Date de publication et mention de la délivrance du brevet: **28.11.2007**
(21) Numéro de dépôt: 05740643.1
(22) Date de dépôt: 09.05.2005
(51) Int. Cl.: A61C 1/18, A61C 1/05, F16L 11/12

(54) **TUYAU ET RACCORD POLYVALENT POUR ALIMENTER DES INSTRUMENTS A USAGE DENTAIRE OU CHIRURGICAL**
ROHR- UND UNIVERSALKUPPLUNG FÜR DIE ZUFUHR VON ZAHNMEDIZINISCHEN ODER CHIRURGISCHEN INSTRUMENTEEN
PIPE AND UNIVERSAL COUPLING FOR SUPPLYING DENTAL OR SURGICAL INSTRUMENTS

(30) Priorité: 28.05.2004 EP 04012687
(43) Date de publication de la demande: 21.02.2007
(73) Titulaire: Bien-Air Holding SA, 2500 Bienne 6 (CH)
(72) Inventeur: MOSIMANN, Vincent, CH-2520 La Neuveville (CH)
(74) Mandataire: Thérond, Gérard Raymond
(86) Numéro de dépôt international: PCT/EP2005/004987
(87) Numéro de publication internationale: WO 2005/115264

(56) Documents cités:
- EP-A- 0 745 358
- US-A1- 2001 031 442
- US-A1- 2003 092 324

## Description

### Arrière-plan de l'invention

La présente invention concerne un tuyau d'alimentation pour relier un instrument à usage dentaire ou chirurgical à une unité d'alimentation et de commande, lequel tuyau contient des conduits de fluide et des lignes électriques et comporte à une extrémité avant un raccord polyvalent pour connecter de manière amovible différents types dudit instrument à une partie ou à l'ensemble desdits conduits et lignes, le raccord polyvalent comportant des connecteurs de fluide raccordés aux dits conduits et des connecteurs électriques raccordés aux dites lignes, chacun desdits connecteurs occupant un emplacement sur une face frontale du raccord polyvalent.

Des tuyaux et raccords de ce genre sont bien connus et sont décrits notamment dans les publications de brevets CH 676 081 et EP 745 358 du même demandeur, l'extrémité arrière du tuyau d'alimentation étant raccordée à une unité d'alimentation et de commande qui peut délivrer, via ce tuyau, différents fluides et différents courants ou signaux électriques pour actionner et/ou commander différents appareils ou instruments pouvant être raccordés au choix à l'extrémité avant du tuyau. A cet effet, cette extrémité avant est pourvue d'un raccord polyvalent qui est aussi appelé "attachement". Celui-ci contient généralement plusieurs connecteurs de fluides et plusieurs connecteurs électriques. Selon le type et la nature de l'instrument devant être raccordé, tous les connecteurs du raccord ou seulement certains d'entre eux seront utilisés et seront donc raccordés à des connecteurs correspondants de l'instrument ou d'un raccord intermédiaire, comme on l'explique notamment dans la demande EP 745 358. C'est pourquoi le raccord ou attachement est qualifié de polyvalent. Des raccords analogues, ayant une autre disposition des connecteurs, sont décrits dans le brevet US 4 080 737.

La figure 1 ci-jointe, reprise du brevet CH 676 081, représente la face frontale du raccord ou attachement polyvalent 4 qui équipe l'extrémité avant du tuyau d'alimentation. Le raccord 4 comporte un corps 5 en matière isolante portant un manchon rotatif fileté 10 qui permet de fixer le raccord 4 d'une manière amovible aux raccords associés. Le corps 5 contient quatre connecteurs 6 à 9 pour des fluides et quatre connecteurs électriques 20 à 23, qui sont arrangés d'une manière standardisée pour pouvoir être utilisés avec des instruments de différents fabricants. Dans l'exemple décrit ici qui se réfère aux produits commercialisés sous la désignation 4VLM par Bien-Air Dental SA, Bienne, Suisse, le connecteur 6 est associé à un conduit qui amène de l'air sous pression à la turbine d'une pièce à main, tandis que le connecteur 7 est associé à un conduit de retour de cet air. Le connecteur 8 est associé à un conduit qui amène de l'eau à l'instrument, tandis que le connecteur 9 est associé à un conduit qui fournit de l'air pulsé à l'instrument, cette eau et cet air pulsé étant destinés à nettoyer, à sécher ou à refroidir le champ de travail. La paire de connecteurs électriques 20 et 21 est destinée à un circuit électrique alimentant une lampe pour éclairer le champ de travail. La paire de connecteurs électriques 22 et 23 sert au circuit d'alimentation d'un moteur électrique d'une pièce à main ou d'un dispositif électrique d'un autre type qu'un moteur ou une lampe, par exemple un appareil de détartrage à ultrasons. Il est connu d'utiliser aussi le circuit d'alimentation passant par les connecteurs 22 et 23 pour transmettre des signaux électriques de commande ou de mesure, sous la forme d'impulsions codées.

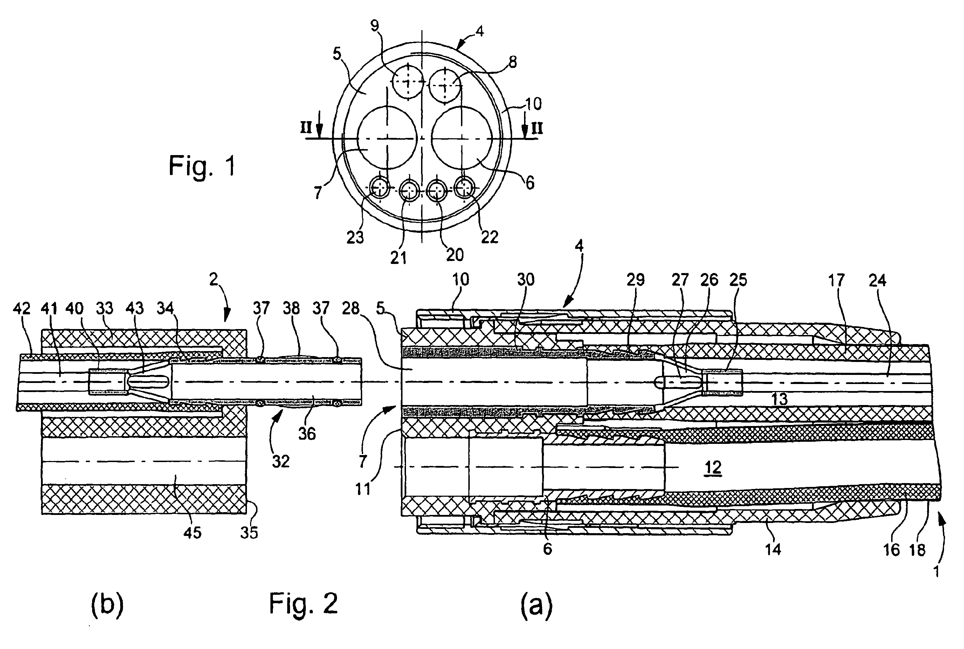

L'agencement représenté à la figure 1 est très largement utilisé dans le monde depuis bien des années, notamment grâce à sa polyvalence et à la standardisation des raccords fournis par les principaux fabricants. Toutefois, cette standardisation présente un inconvénient dans certains cas, par le fait qu'il devient presque impossible d'installer des connecteurs supplémentaires dans un tel raccord, quasiment tous les emplacements disponibles sur la face frontale étant occupés par les connecteurs habituels. Or, avec l'évolution de la technique et en particulier des pièces à main à usage dentaire ou chirurgical, des liaisons supplémentaires ou différentes entre la pièce à main et l'unité d'alimentation et de commande deviennent souhaitables ou nécessaires, et il serait utile de les réaliser sans devoir modifier la plupart des raccords des instruments que les praticiens utilisent actuellement. Un exemple typique d'une telle liaison supplémentaire est la mise à terre de certains appareils ou instruments dont les circuits électriques pourraient présenter un risque pour un patient. Cette mise à terre, obligatoire dans certains cas, devrait utiliser l'un des quatre connecteurs 20 à 23 dans l'exemple de la figure 1 et pourrait donc conduire à la suppression d'un des circuits électriques. Il existe donc le besoin d'une autre solution à ce problème.

Dans d'autres domaines de la technique, des circuits conducteurs ont été incorporés à des tuyaux de fluide et à leurs connecteurs afin de former une ligne de mise à terre. Par exemple, une solution représentée aux figures 6 à 8 de la demande de brevet EP 1 310 721 utilise, pour dissiper d'éventuelles charges électrostatiques, des tuyaux ayant une couche conductrice et des connecteurs ayant des pièces de contact situées à l'intérieur du conduit de fluide.

Un autre exemple se trouve dans le brevet US 1 890 290, qui décrit des raccords pour des tuyaux de pompe à incendie dont les parois contiennent une paire de fils conducteurs formant un circuit de transmission d'un signal électrique. Le raccordement d'un des fils s'effectue directement par les bagues métalliques filetées des raccords, tandis que la liaison du deuxième fil s'effectue par l'intermédiaire de pièces de contact disposées dans le conduit de fluide. Cet agencement semble difficilement utilisable d'une manière sûre, si l'on considère qu'au moins une des deux lignes électriques devrait être bien isolée sur toute sa longueur par rapport au milieu extérieur et à l'eau contenue dans le tuyau.

### Résumé de l'invention

La présente invention a pour objet un agencement d'un tuyau d'alimentation, en particulier de son raccord polyvalent, qui permet d'augmenter le nombre de liaisons électriques afin d'offrir d'autres possibilités de combinaison de lignes et conduits d'alimentation ou de commande et donc permettre de connecter au besoin de nouveaux types d'instruments dentaires ou chirurgicaux, sans nécessairement changer la disposition habituelle des connecteurs dans le raccord polyvalent ni les dimensions de celui-ci.

A cet effet, il est prévu un tuyau d'alimentation du genre énoncé en préambule ci-dessus, caractérisé en ce qu'il contient au moins une ligne électrique supplémentaire comportant un conducteur supplémentaire qui n'est pas raccordé à l'un desdits connecteurs électriques, mais à l'un des connecteurs de fluide du raccord polyvalent, ce connecteur dit à double fonction étant agencé pour assurer au choix une connexion de fluide, une connexion électrique ou les deux.

Ainsi, non seulement l'invention augmente effectivement le nombre de liaisons électriques disponibles en permettant de conserver la disposition et la taille du raccord polyvalent, mais en outre elle multiplie le nombre d'agencements différents de connecteurs dans les raccords à brancher à ce raccord polyvalent, en particulier les raccords propres aux divers instruments qu'on peut envisager d'alimenter par le tuyau et l'unité associée.

Dans un première forme de réalisation, le connecteur à double fonction comporte une partie métallique tubulaire reliée électriquement au conducteur supplémentaire et raccordée à un tube flexible s'étendant dans le tuyau, pour former avec ce tube un conduit de fluide, ladite partie tubulaire formant à la fois un organe de contact électrique et un organe d'emboîtement pour coopérer avec une partie tubulaire d'un connecteur faisant partie d'un second raccord relié audit instrument. Grâce à l'invention, ce second raccord, destiné à être couplé de manière amovible au raccord polyvalent, peut avoir des configurations plus variées que selon l'art antérieur, puisque pour coopérer avec chaque connecteur à double fonction du raccord polyvalent, il peut comporter à l'emplacement correspondant soit un connecteur de fluide, soit un connecteur électrique, soit un connecteur à double fonction.

Dans une autre forme de réalisation, le connecteur à double fonction peut comporter un alésage cylindrique, ménagé dans un corps isolant du raccord polyvalent, et un élément de contact mâle, femelle ou hermaphrodite, disposé au centre dudit alésage cylindrique et relié électriquement au conducteur supplémentaire.

### Description sommaire des dessins

La figure 1 représente la face frontale d'un raccord connu d'un tuyau d'alimentation d'instruments dentaires, dans lequel la présente invention est applicable.
La figure 2 représente schématiquement un premier mode de réalisation de l'invention, la partie (a) de la figure étant une vue en coupe longitudinale d'un raccord femelle monté sur le tuyau d'alimentation, par exemple suivant la ligne II-II de la figure 1, tandis que la partie (b) de la figure est une vue en coupe longitudinale d'un raccord mâle agencé pour coopérer avec le raccord femelle.
La figure 3 représente schématiquement un deuxième mode de réalisation dans la même application que la figure 2, par une vue en coupe longitudinale partielle représentant un raccord mâle en (a) et un raccord femelle en (b).
La figure 4 est une vue analogue à la figure 3 et représente schématiquement un troisième mode de réalisation.
La figure 5 est une vue analogue à la figure 3 et représente schématiquement un quatrième mode de réalisation.

### Description détaillée de divers modes de réalisation de l'invention

Un premier mode de réalisation sera décrit à titre d'exemple en référence à la figure 2, dont la partie (a) montre un raccord polyvalent 4 monté sur l'extrémité avant d'un tuyau d'alimentation 1 provenant d'une unité d'alimentation et de commande, tandis que la partie (b) montre schématiquement une partie d'un raccord arrière 2 agencé pour se coupler au raccord 4. Le raccord arrière 2 peut faire partie d'une pièce intermédiaire ou d'une pièce à main, ou encore d'un autre appareil alimenté et/ou commandé via le tuyau 1, par exemple un appareil comportant un moteur électrique ou une turbine à air.

Le raccord 4 est analogue à celui de la figure 1 décrite plus haut, mais modifié par l'adjonction d'une ligne électrique supplémentaire, en l'occurrence une ligne de mise à terre. On distingue dans le dessin son corps 5 en matière synthétique isolante, contenant notamment le connecteur femelle 6 pour un conduit 12 d'air sous pression et le connecteur femelle 7 pour un conduit 13 de retour d'air. Le corps 5 est monté dans un fourreau 14 qui porte le manchon rotatif 10 et dont l'extrémité arrière entoure le tuyau d'alimentation 1. Celui-ci comprend quatre tubes flexibles pour les fluides, à savoir un tube 16 emboîté sur l'arrière du connecteur 6, un tube 17 emboîté sur l'arrière du connecteur 7 et deux autres tubes raccordés aux connecteurs de fluide 8 et 9, et en outre quatre lignes électriques isolées raccordées respectivement aux connecteurs électriques 20 à 23. Le tuyau 1 comporte de préférence une gaine souple 18 enveloppant l'ensemble des tubes et lignes.

Dans le tuyau d'alimentation 1, la ligne de mise à terre est formée par un conducteur flexible 24 qui s'étend librement à l'intérieur du tube de retour d'air 17, par exemple une tresse de fil de cuivre sans isolation. Bien entendu, on pourrait utiliser un conducteur isolé si la ligne en question n'était pas une ligne de mise à terre ou si le conduit où elle se trouve pouvait contenir un fluide conducteur. Dans le cas présent, on a choisi de placer la ligne de terre dans le tube 17 parce que c'est le plus gros, si bien que la réduction de la section de passage de l'air est proportionnellement la plus petite. Le conducteur 24 est raccordé mécaniquement et électriquement, par exemple par sertissage et/ou soudage, à l'extrémité arrière du connecteur 7 qui s'étend sous forme d'une petite douille centrale 25 à l'intérieur du conduit d'air 13. Une partie conique 26 munie de plusieurs orifices 27 pour le passage de l'air raccorde la douille 25 à la partie principale tubulaire 30 du connecteur 7, ayant un alésage cylindrique 28 qui débouche sur la face frontale 11 du raccord 4. Le connecteur 7 est évidemment fait d'un matériau bon conducteur de l'électricité, de préférence un métal, et la surface extérieure 29 de sa partie tubulaire 30 est emboîtée de manière étanche dans le tube 17, si bien qu'il peut servir en même temps ou alternativement de connecteur d'air et/ou de connecteur électrique, d'une façon qui n'exige pas d'emplacement supplémentaire au niveau de la face frontale 11 du raccord 4.

Etant donné que la ligne de terre comprenant le conducteur 24 et le connecteur 7 se trouve toujours au potentiel électrique zéro par rapport à la terre, ses composants n'ont pas besoin d'isolation électrique et peuvent donc être en contact avec l'air de retour qui passe dans le conduit 13 et qui peut être chargé de gouttelettes d'eau aspirées sur le champ de travail. Toutefois la ligne de terre peut évidemment être combinée à un autre des tubes du tuyau 1, y compris un tube conduisant un fluide conducteur tel que de l'eau.

Dans cet exemple, le connecteur 6 du conduit d'air comprimé 12 est réalisé de la façon traditionnelle, mais bien entendu il pourrait aussi être adapté à une double fonction pneumatique et électrique comme le connecteur 7, en combinaison avec une ligne électrique de commande ou d'alimentation passant dans le tube 16 où l'air comprimé est sec, comme le fait le conducteur 24 dans l'autre tube. On disposerait ainsi d'un nouveau circuit électrique comprenant deux lignes dans le tuyau 1. Cependant, le connecteur 6 devrait alors être isolé.

Dans la partie (b) de la figure 2, seuls les éléments du raccord 2 qui sont propres à la mise en oeuvre de la présente invention sont représentés. Pour coopérer avec le connecteur femelle 7, un connecteur mâle 32 à double fonction pneumatique et électrique est monté dans le corps 33 du raccord et comporte une partie tubulaire 34 qui dépasse la face frontale 35 de ce corps afin qu'on puisse l'enficher dans l'alésage 28 du connecteur 7 et raccorder ainsi son alésage intérieur 36 au conduit de retour d'air 13. Pour coopérer avec la surface de l'alésage 28, la surface extérieure de la partie 34 est pourvue de joints toriques d'étanchéité 36 et de saillies 37 assurant un bon contact électrique. L'autre extrémité du connecteur 32, située du côté considéré ici comme l'avant, peut être réalisée de manière semblable à celle du connecteur 7, avec une petite douille centrale 40 fixée sur l'extrémité d'un conducteur 41 s'étendant dans un tube 42 qui conduit l'air en retour de l'instrument raccordé. Une partie conique ajourée 43 solidarise la douille 40 et la partie tubulaire 34 du connecteur mâle, qui est de préférence en métal.

Dans le connecteur 7, la position de la douille 25 et de la partie conique 26 au-delà de l'extrémité arrière de la partie tubulaire 30 a deux avantages. D'une part, cela permet de sertir et ou souder aisément la douille 25 sur le conducteur 24, et d'autre part les éléments 25 et 26 n'interfèrent pas avec le connecteur mâle 32 se logeant dans la partie tubulaire 30. Cette dernière n'est donc pas plus longue que dans l'exécution traditionnelle selon la figure 1, c'est-à-dire que l'invention n'impose pas d'allonger le raccord polyvalent 4.

Le raccord 2 comporte en outre d'autres connecteurs mâles (ou femelles ou hermaphrodites selon les cas) pour coopérer avec l'ensemble ou une partie des connecteurs du raccord polyvalent 4, selon les besoins de l'instrument raccordé. Si par exemple le connecteur femelle 6 était à double fonction pneumatique et électrique comme le connecteur 7, un connecteur mâle correspondant, semblable au connecteur 32, pourrait être monté dans un canal 45 du corps 33.

Dans les instruments dentaires actuels ayant un raccord du genre du raccord 2, le corps 33 est en général métallique. Il peut aussi l'être dans le présent exemple dans la mesure où la ligne électrique passant par le connecteur 32 est une ligne de mise à terre. Par contre, le corps 33 devrait être fait d'un matériau isolant si le connecteur 32 ou un autre connecteur similaire disposé dans le raccord 2 faisait partie d'une ligne électrique à potentiel non nul.

Au vu de la description qui précède, on comprendra sans peine que selon le type d'instrument à alimenter via le tuyau 1, le connecteur à double fonction 7 peut recevoir au choix un connecteur à double fonction pneumatique et électrique, tel que le connecteur 32, ou un connecteur pneumatique seulement comme c'est le cas dans l'art antérieur, ou un connecteur électrique seulement, s'il s'agit d'alimenter un instrument n'ayant pas besoin d'un retour d'air, par exemple un instrument chirurgical à moteur électrique. La polyvalence du raccord 4 est ainsi accrue.

On notera que dans la plupart des cas, la ligne électrique supplémentaire passant par le connecteur à double fonction pourra être utilisée, au choix, pour transmettre des signaux et/ou une puissance d'alimentation électrique.

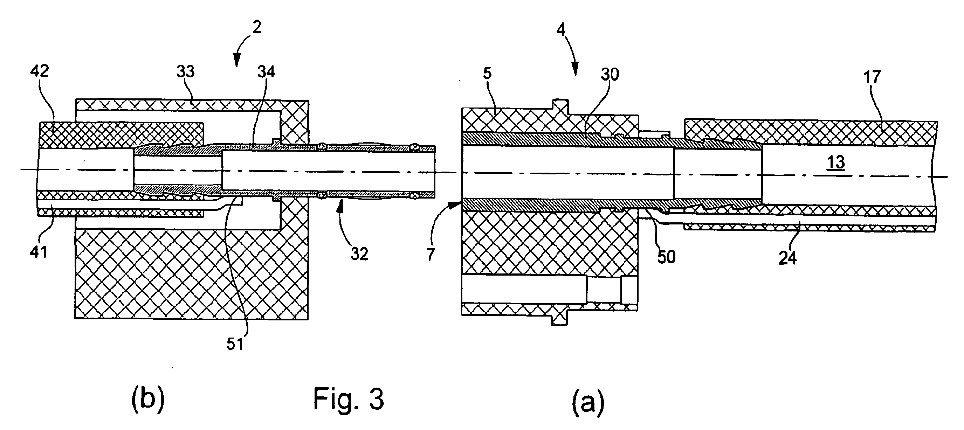

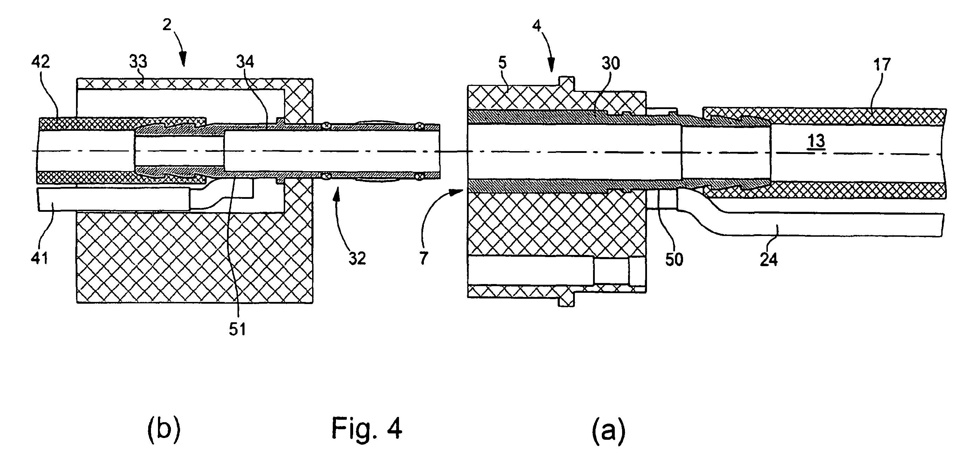

Les figures 3 et 4 illustrent deux autres modes de réalisation qui ne diffèrent substantiellement du premier que par la situation de la ligne électrique supplémentaire par rapport au conduit de fluide associé. C'est pourquoi les dessins sont simplifiés et on ne décrira que ces différences ci-dessous.

Dans la réalisation selon la figure 3, les conducteurs 24 et 41 sont noyés dans l'épaisseur de la paroi des tubes correspondants 17 et 42 au lieu de se trouver dans le conduit d'air 13. Leurs extrémités respectives sont soudées en 50, 51 à la surface extérieure de la partie tubulaire 30, 34 du connecteur associé. Un tel conducteur peut se présenter sous la forme d'une bande métallique flexible ou d'une tresse aplatie et être incorporé à la paroi lors de la fabrication du tube par extrusion. On remarque que les conducteurs 24 et 41 sont ainsi maintenus à l'abri des fluides circulant dans le tuyau d'alimentation, ce qui peut éviter d'éventuels problèmes de corrosion. En outre, ils ne réduisent pas la section de passage de l'air dans le conduit 13.

Dans la réalisation selon la figure 4, les conducteurs 24 et 41 sont placés en dehors des tubes correspondants 17 et 42 et ils ont de préférence une gaine isolante. Leurs extrémités respectives sont dénudées et soudées en 50, 51 à la surface extérieure de la partie tubulaire 30, 34 du connecteur associé comme dans l'exemple de la figure 3. Le conducteur isolé 24 est logé dans le tuyau d'alimentation 1 de la même manière que les conducteurs électriques habituels raccordés aux connecteurs 20 à 23, de sorte qu'il peut aussi être raccordé de la même manière que ceux-ci à l'unité d'alimentation et de commande.

On notera que n'importe lequel des connecteurs mâles 32 des figures 2b, 3b et 4b peut être enfiché dans n'importe lequel des connecteurs femelles 7 des figures 2a, 3a et 4a. Autrement dit, la disposition du conducteur supplémentaire n'a pas besoin d'être la même dans les deux raccords accouplés 2 et 4.

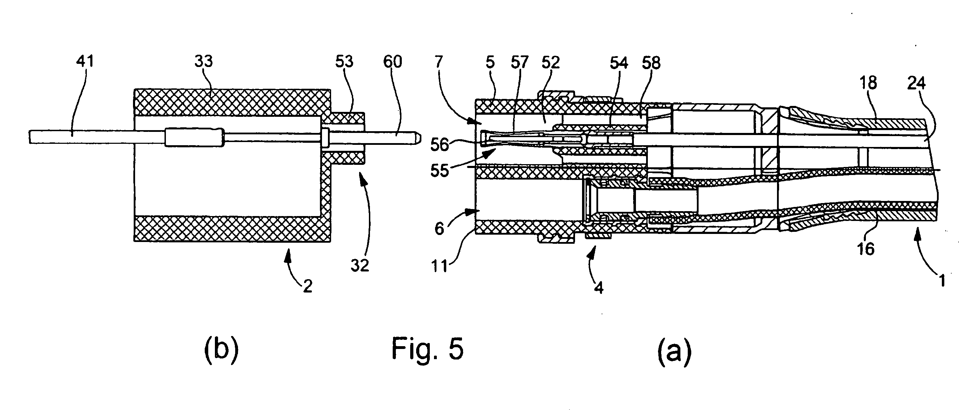

La figure 5 représente de manière partielle et schématique une variante de la construction selon la figure 2, se distinguant par un agencement différent des organes de contact électrique. En outre, dans cet exemple le tube de retour d'air 17 de la figure 2 est supprimé, le tuyau d'alimentation 1 étant prévu dans ce cas pour des instruments qui n'ont pas besoin d'un conduit de retour d'air, en particulier des pièces à main à moteur électrique. Par contre, ces pièces à main peuvent avoir besoin d'une ligne électrique supplémentaire pour transmettre des signaux de mesure ou de commande, par exemple un signal indiquant le couple produit par le moteur, ou pour transmettre un courant afin d'alimenter le moteur ou un autre organe. Cette ligne est formée par les conducteurs supplémentaires 24 et 41 décrits dans les exemples précédents et reliés mutuellement par le connecteur femelle 7 et le connecteur mâle 32.

Le connecteur femelle 7 comporte un alésage cylindrique 52 ménagé dans le corps isolant 5 et dans lequel peut s'emboîter un embout cylindrique 53 du connecteur mâle 2. A l'intérieur de l'alésage 52, le corps 5 comporte un support évidé 54 portant au centre de l'alésage 52 une douille métallique de contact 55, dont la partie avant est munie de fentes 56 délimitant des lamelles élastiques 57. Le support évidé 54 comporte des canaux 58 pour le passage de l'air, le cas échéant. L'extrémité avant de la douille 55 se trouve de préférence en retrait de la face frontale 11 du corps isolant. L'extrémité du conducteur 24 est dégarnie de sa gaine isolante et fixée dans la partie arrière de la douille 55. Dans ce cas où il n'y a pas de tube de retour d'air dans le tuyau d'alimentation 1, le conducteur 24 est logé simplement à l'intérieur de la gaine 18 du tuyau, où il peut être groupé avec les autres lignes électriques.

Dans le connecteur mâle 32, le conducteur 41 est raccordé à une broche de contact 60 supportée par un élément non représenté du corps isolant 33 du raccord 2. Cette broche se trouve au centre du conduit formé par l'embout 53 et, lorsqu'on accouple les deux raccords 2 et 4, elle se fiche dans la douille 55 pour assurer la connexion électrique entre les conducteurs 24 et 41.

Bien entendu, le système de connexion électrique à broche et douille représenté à la figure 5 peut aussi être intégré au conduit de retour d'air 13 représenté à la figure 2 et formé par les tubes 17 et 42, ou à un autre des connecteurs à double fonction des raccords 2 et 4 décrits plus haut.

## Revendications

1. Tuyau d'alimentation pour relier un instrument à usage dentaire ou chirurgical à une unité d'alimentation et de commande, lequel tuyau contient des conduits de fluide et des lignes électriques et comporte à une extrémité avant un raccord polyvalent (4) pour connecter de manière amovible différents types dudit instrument à une partie ou à l'ensemble desdits conduits et lignes, le raccord polyvalent comportant des connecteurs de fluide (6 à 9) raccordés aux dits conduits et des connecteurs électriques (20 à 23) raccordés aux dites lignes, chacun desdits connecteurs occupant un emplacement sur une face frontale (11) du raccord polyvalent,
le tuyau d'alimentation (1) étant **caractérisé en ce qu'**il contient au moins une ligne électrique supplémentaire comportant un conducteur supplémentaire (24) qui n'est pas raccordé à l'un desdits connecteurs électriques (20 à 23), mais à l'un (7) des connecteurs de fluide du raccord polyvalent (4), ce connecteur dit à double fonction étant agencé pour assurer au choix une connexion de fluide, une connexion électrique ou les deux.

2. Tuyau d'alimentation selon la revendication 1, **caractérisé en ce que** le connecteur à double fonction (7) comporte une partie métallique tubulaire (30) reliée électriquement au conducteur supplémentaire (24) et raccordée à un tube flexible (17) s'étendant dans le tuyau, pour former avec ce tube un conduit de fluide (13), ladite partie tubulaire formant à la fois un organe de contact électrique et un organe d'emboîtement pour coopérer avec une partie tubulaire (32) d'un connecteur relié audit instrument.

3. Tuyau d'alimentation selon la revendication 2, **caractérisé en ce que** le conducteur supplémentaire (24) est disposé dans le conduit de fluide (13) à l'intérieur dudit tube (17).

4. Tuyau d'alimentation selon la revendication 3, **caractérisé en ce que** le conducteur supplémentaire (24) est raccordé à une douille métallique (25) solidaire de ladite partie tubulaire (30) et située dans le conduit de fluide (13).

5. Tuyau d'alimentation selon la revendication 2, **caractérisé en ce que** le conducteur supplémentaire (24) est disposé dans la paroi dudit tube (17).

6. Tuyau d'alimentation selon la revendication 2, **caractérisé en ce que** le conducteur supplémentaire (24) est disposé à l'extérieur dudit tube (17).

7. Tuyau d'alimentation selon l'une des revendications précédentes, **caractérisé en ce que** le conduit associé au connecteur à double fonction (7) est un conduit d'air (13).

8. Tuyau d'alimentation selon la revendication 1, **caractérisé en ce que** le connecteur à double fonction (7) comporte un alésage cylindrique (52), ménagé dans un corps isolant (5) du raccord polyvalent (4), et un élément de contact (55) disposé au centre dudit alésage cylindrique et relié électriquement au conducteur supplémentaire (24).

## Claims

1. Supply pipe for connecting an instrument for dental or surgical use to a control and supply unit, said pipe containing fluid conduits and electric lines and including at a front end a universal coupling (4) for connecting different types of said instrument in a removable manner to part or all of said conduits and lines, the universal coupling including fluid connectors (6 to 9) coupled to said conduits and electric connectors (20 to 23) coupled to said lines, each of said connectors occupying a place on a front face (11) of the universal coupling,
said supply pipe (1) being **characterized in that** it contains at least one additional electric line including an additional conductor (24) which is not coupled to one of said electric connectors (20 to 23), but to one (7) of the fluid connectors of the universal coupling (4), said dual function connector being arranged to provide a choice between a fluid connection, an electric connection or both.

2. Supply pipe according to claim 1, **characterized in that** the dual function connector (7) includes a tubular metal part (30) electrically connected to the additional conductor (24) and coupled to a flexible tube (17) extending into the pipe, to form with said tube a fluid conduit (13), said tubular part forming both an electric contact member and a fit member for cooperating with a tubular part (32) of a connector connected to said instrument.

3. Supply pipe according to claim 2, **characterized in that** the additional conductor (24) is arranged in the fluid conduit (13) inside said tube (17).

4. Supply pipe according to claim 3, **characterized in that** the additional conductor (24) is coupled to a metal sleeve (25) secured to said tubular part (30) and located in the fluid conduit (13).

5. Supply pipe according to claim 2, **characterized in that** the additional conductor (24) is arranged in the wall of said tube (17).

6. Supply pipe according to claim 2, **characterized in that** the additional conductor (24) is arranged outside said tube (17).

7. Supply pipe according to any of the preceding claims, **characterized in that** the conduit associated with the dual function connector (7) is an air conduit (13).

8. Supply pipe according to claim 1, **characterized in that** the dual function connector (7) includes a cylindrical bore (52) arranged in an insulating body (5) of the universal coupling (4), and a contact element (55) arranged at the centre of said cylindrical bore and electrically connected to the additional conductor (24).

## Patentansprüche

1. Versorgungsschlauch, um ein Instrument für zahnärztliche oder chirurgische Verwendung mit einer Versorgungs- und Steuereinheit zu verbinden, wobei der Schlauch Fluidrohrleitungen und elektrische Leitungen enthält und an einem vorderen Ende ein universelles Verbindungselement (4) zum lösbaren Verbinden verschiedener Typen des Instruments mit einem Teil der oder mit allen Rohrleitungen und Leitungen umfasst, wobei das universelle Verbindungselement Fluidverbinder (6 bis 9), die mit den Rohrleitungen verbunden sind, und elektrische Verbinder (20 bis 23), die mit den Leitungen verbunden sind, umfasst, wobei jeder der Verbinder an einer Vorderseite (11) des universellen Verbindungselements eine bestimmte Stelle einnimmt,
wobei der Versorgungsschlauch (1) **dadurch gekennzeichnet ist, dass** er wenigstens eine zusätzliche elektrische Leitung enthält, die einen zusätzlichen Leiter (24) umfasst, der nicht mit einem der elektrischen Verbinder (20 bis 23), sondern mit einem (7) der Fluidverbinder des universellen Verbindungselements (4) verbunden ist, wobei dieser so genannte Doppelfunktionsverbinder so beschaffen ist, dass er wahlweise eine Fluidverbindung, eine elektrische Verbindung oder beides gewährleistet.

2. Versorgungsschlauch nach Anspruch 1, **dadurch gekennzeichnet, dass** der Doppelfunktionsverbinder (7) ein rohrförmiges metallisches Teil (30), das mit dem zusätzlichen Leiter (24) elektrisch verbunden und mit einem flexiblen Rohr (17), das durch den Schlauch verläuft, verbunden ist, um mit diesem Rohr eine Fluidrohrleitung (13) zu bilden, wobei das rohrförmige Teil sowohl ein elektrisches Kontaktorgan als auch ein Umschließungsorgan, um mit einem rohrförmigen Teil (32) eines mit dem Instrument verbundenen Verbinders zusammenzuwirken, bildet.

3. Versorgungsschlauch nach Anspruch 2, **dadurch gekennzeichnet, dass** der zusätzliche Leiter (24) in der Fluidrohrleitung (13) innerhalb des Rohrs (17) angeordnet ist.

4. Versorgungsschlauch nach Anspruch 3, **dadurch gekennzeichnet, dass** der zusätzliche Leiter (24) mit einer metallischen Hülse (25) verbunden ist, die mit dem rohrfömigen Teil (30) fest verbunden ist und sich in der Fluidrohrleitung (13) befindet.

5. Versorgungsschlauch nach Anspruch 2, **dadurch gekennzeichnet, dass** der zusätzliche Leiter (24) in der Wand des Rohrs (17) angeordnet ist.

6. Versorgungsschlauch nach Anspruch 2, **dadurch gekennzeichnet, dass** der zusätzliche Verbinder (24) außerhalb des Rohrs (17) angeordnet ist.

7. Versorgungsschlauch nach einem der vorhergehenden Ansprüche, **dadurch gekennzeichnet, dass** die Rohrleitung, die dem Doppelfunktionsverbinder (7) zugeordnet ist, eine Luftrohrleitung (13) ist.

8. Versorgungsschlauch nach Anspruch 1, **dadurch gekennzeichnet, dass** der Doppelfunktionsverbinder (7) eine zylindrische Bohrung (52), die in einem isolierenden Körper (5) des universellen Verbindungselements (4) ausgebildet ist, und ein Kontaktelement (55), das im Zentrum der zylindrischen Bohrung angeordnet und mit dem zusätzlichen Leiter (24) elektrisch verbunden ist, umfasst.
